# EUROPEAN PATENT SPECIFICATION

(11) **EP 4 419 188 B1**
(45) Date of publication and mention of the grant of the patent: **23.04.2025**
(21) Application number: 22793243.1
(22) Date of filing: 14.10.2022
(51) Int. Cl.: A61N 1/04, A61N 1/32, A61N 1/36, A61N 1/372

(54) **BIOMEDICAL DEVICE FOR THE TREATMENT OF ULCERS OR LESIONS**
BIOMEDIZINISCHE VORRICHTUNG ZUR BEHANDLUNG VON GESCHWÜREN ODER LÄSIONEN
DISPOSITIF BIOMÉDICAL POUR LE TRAITEMENT D'ULCÈRES OU DE LÉSIONS

(30) Priority: 18.10.2021 IT 202100026615
(43) Date of publication of application: 28.08.2024
(73) Proprietor: Fremslife S.r.l., 16149 Genova (IT); Fondazione Istituto Italiano di Tecnologia, 16163 Genova (IT)
(72) Inventor: BERTORA, Franco, 16153 Genova (IT); PALERMO, Michele, 16153 Genova (IT); MIRACOLI, Luigi, 16153 Genova (IT); CONTARDI, Marco, 90147 Palermo (IT); BERTORELLI, Rosalia, 20125 Milano (IT); SUMMA, Maria, 15067 Novi Ligure (IT); ATHANASSIOU, Athanassia, 16014 Ceranesi (IT)
(74) Representative: Karaghiosoff, Giorgio Alessandro
(86) International application number: PCT/IB2022/059855
(87) International publication number: WO 2023/067449

(56) References cited:
- WO-A1-2013/155193
- WO-A1-2019/166965
- US-A1- 2007 179 585
- US-A1- 2018 311 499
- US-A1- 2019 098 738
- US-A1- 2019 240 475
- US-A1- 2021 290 942

## Description

The present invention relates to a biomedical device for the treatment of ulcers or lesions, particularly for the treatment of diabetic ulcers.

Diabetes is considered to be the most critical global noncontaqious disease. There are several scientific opinions relating to this disease. While there is no doubt that it is a metabolic disorder characterized by hyperglycemia, on the other hand the onset of diabetes is accompanied by other factors, such as obesity and cardiovascular disorders. In any case, this disease provides a significant socio-economic burden since it is a chronic disease, which must be kept under control for the entire life of patients and affects millions of people around the world.

If diagnosed late, due to a delay in the actions to control the glycemic level compared to the time of the onset of the disease, this provides effects that deeply compromise the functionality of the patients' feet and leqs, causing severe neuropathic pain, circulation problems and the formation of chronic ulcers in these areas of the body. The progression of all these factors can also lead to the amputation of part of the leq/foot.

Diabetic ulcers are characterized by a chronic inflammatory environment and an altered or interrupted skin regeneration mechanism. These unresolved wounds are a gateway for pathogenic microorganisms due to the lack of skin tissue. Furthermore, wound exudate is a perfect medium for bacterial proliferation and as a result, the difficult management of the infection makes the treatment of diabetic ulcers a major concern.

The condition of persistent hyperglycemia, typical of diabetes, deeply affects the lower limbs causing circulation problems, poor blood circulation, peripheral neuropathic pain, and loss of skin sensitivity, leading to tissue death (maceration, gangrene).

Pharmacological treatments are known to the state of the art, which include wound dressings, which are designed to control and support the healing of ulcers locally. The dressings are made in the form of films, fibrous mats, sponges and hydrogels for the *in situ* administration of antibiotics, antioxidant and anti-inflammatory molecules and growth factors. The modern biomaterials used in these dressing devices try, at the same time, to counteract the invasion and proliferation of microorganisms, eliminate free radicals present in the exudate, rebalance the persistent inflammatory condition and promote the proliferation of skin cells in order to achieve proper wound repair.

However, the low scalability, high production cost, local side effects and bacterial resistance still deeply limit the introduction of these technologies. Furthermore, the dressings are not designed to deal with neuropathic pain and the circulation problem related to diabetic ulcers, completely limiting their use to a local application, and making the results, in terms of healing effectiveness, relatively limited also in relation to the therapy duration. The therapeutic process that makes use of this kind of dressings requires interventions by healthcare personnel who, in addition to performing the replacement of the device, also control the evolution of the pathological condition and/or direct interventions on the ulcer itself, such as for instance cleaning, extemporaneous application of further drugs and other actions. However, this requires a constant and cyclical intervention of the healthcare personnel which is not limited to a mere control activity, so that the treatment costs become relatively high.

In the state of the art, biomedical devices for therapeutic applications by electrical stimulation are also known.

WO2019166965A1 discloses an electrical stimulation apparatus with pulse generators and electrodes on flexible supports for independent channel treatment, featuring control units and interfaces, adaptable for drug administration.

US20180311499A1 discloses a method for treating dermatological conditions using neuromodulatory agents, including pharmacological and electrical stimuli, targeting conditions like psoriasis and ulcers with potential implantable devices.

In general, these are devices that provide for the emission through the skin, by means of electrodes applied to the skin itself, of electrical impulses configured according to particular sequences and according to particular parameters such as signal power, voltage, current intensity, frequencies and waveforms. They are therefore non-invasive therapeutic biomedical devices that show efficacy in the treatment of neuropathic pain, in the improvement of circulatory flow and in the treatment of vascular disorders related to diabetes as well as in the treatment of skin lesions such as ulcers related to diabetes.

Furthermore, current electrostimulation techniques used in the treatment of diabetic ulcers require a relatively long treatment time, which causes patient discomfort and increases treatment costs.

Particularly, electrostimulation devices operating according to FREMS technology have proved effective both in the treatment of neuropathic pain and in increasing blood flow in the anatomical areas where they are applied. Studies and experiments related to this technology show an ability to increase the release of growth factors such as vascular endothelial growth factor (VEGF), valid for the treatment of diabetic ulcer.

Frequency and amplitude modulated neuronal electrical stimulation or FREMS ~ (Frequency Rhythmic Electric Modulation is described in documents IT 1341343 (2007), WO2004067087A1, EP1305080 and WO 2004/084988 (incorporated herein by reference) and is characterized by the use of transcutaneous electrical currents, produced by sequential electrical impulses with variable frequency and duration. The frequency can vary from 0.1 to 999 Hz, the duration of the stimulus is between 0.1 and 40 µs and the voltage, constantly maintained above the perceptual threshold, is comprised between 0.1 and 300 V (preferably 150 V).

Particularly, WO 2004/084988 describes a microcirculation activation sequence (ATMC) and a muscle fiber decontracting sequence (DCTR), which are capable of soliciting various functional districts, including striated muscle, smooth muscle and peripheral nervous system. The aforementioned stimulation sequences are based on three fundamental parameters: the duration of the stimulus, the frequency of the stimulus and the time intervals during which different duration/frequency combinations follow one another. The operating general pattern of the stimulation sequences mirrors the digital-to-analogic transduction that occurs in the transmission of a nerve impulse.

Frequency and amplitude modulated neuronal electrical stimulation or FREMS ~ (Frequency Rhythmic Electric Modulation described in the aforementioned WO 2004/084988 and in WO 2004/067087 (incorporated herein by reference), is characterized by the use of transcutaneous electrical currents, produced by sequential electrical impulses with variable frequency and duration. The frequency can vary from 0.1 to 999 Hz, the duration of the stimulus is between 0.1 and 40 µs and the voltage, kept constantly above the perceptual threshold, is between 0.1 and 300 V (preferably 150 V). By suitably combining the aforementioned variations in frequency and duration, a specific sequence is obtained, called DCTR, having a decontracting effect and including a series of sub-phases, called A, B and C. Frequency and amplitude are constant in sub-phase A, the frequency is constant and the amplitude variable in sub-phase B, the frequency is variable and the amplitude constant in sub-phase C.

Experimental studies have made it possible to evaluate the effects of FREMS and the ability of the latter to evoke composed muscle action potentials (cMAP), obtainable in the proper adductor muscle of the big toe by stimulating the posterior tibial nerve, as well as the variation in amplitude of the reflex H using the latter as a conditioning stimulus. As described in WO 2004/084988, the aforementioned experimental studies have also shown that the greater amplitude of the cMAPs (0.60 ± 0.02 mV) obtainable is about 15 times lower than that of the cMAPs obtained with the known devices delivering TENS currents, i.e. of the order of 9 ± 0.6 mV with stimuli having a duration typically included in a range of 200-1000 µs. It has also been observed that the maximum amplitude value of the cMAPs is obtained in the presence of a duration/frequency ratio equal to 0.13 (40µs/29Hz).

A further kind of sequence, called ATCM and suitably designed to obtain a vasoactive effect, has a prevalent action on the motility of the microcirculation, that is, of the smooth sphincters of the arterioles and venules of the subcutaneous tissue. In practice, as described in WO 2004/084988, a system is obtained which produces a sequence of vasodilatations and vasoconstrictions with sequential increases and decreases in the blood flow of the microcirculation surrounding the stimulation zone. These vasodilatations and vasoconstrictions produce a "pump" effect evidently produced by a neuromodulation of the sympathetic autonomic system, which influences the vasoaction precisely through the smooth muscles of the capillaries and arterioles. In this way it can be seen that this subsequence, characterized by alternating variations of the rheobase, therefore produces a vasoactive effect consisting of phases of vasodilatation and sequential vasoconstriction phases. This certainly also produces a draining effect and, above all, an elasticization of the microcirculation and a modulation of the latter around a main bearing event that determines its average variation.

Despite the aforementioned clinical effects, even the biomedical therapeutic devices operating according to the FREMS technology lack some crucial aspects to support the wound healing process. For instance, this system does not have antibacterial and antifungal properties, cannot guarantee any covering action of the wound and the ability to absorb exudate. Furthermore, investigations regarding the anti-inflammatory effects for FREMS technology have not yet been reported. Therefore, both for devices operating according to Frems technology, and for electrostimulation devices operating according to other stimulation technologies or methodologies, as regards the configuration of the pulse sequences transmitted to the anatomical districts, the effectiveness remains limited and not completely decisive for achieving the healing of ulcers, particularly diabetic ulcers and particularly as regards the healing times and the level of healing of the lesions.

The invention is defined in the appended claims. Aspects, embodiments and examples disclosed herein which do not fall within the scope of the appended claims do not form part of the invention, and are merely provided for illustrative purposes.

An aspect of the present invention is therefore that of producing a kit comprising biomedical device for the treatment of ulcers, particularly for the treatment of diabetic ulcers, which has an improved therapeutic efficacy as regards the times and levels of healing of ulcers and particularly of diabetic ulcers.

A further aspect relates to the production of a biomedical device for the treatment of ulcers and particularly of diabetic ulcers which in combination with an improved therapeutic efficacy presents a greater convenience of application *in situ* and/or replacement during the therapeutic process and which is also relatively compact, easy to control and use and relatively inexpensive especially with respect to currently known devices.

Sodium alginate is a natural carbohydrate polymer used in the wound healing process such as known from Varaprasad et al. 2020 "Alginate-based composite materials for wound dressing application: a mini review"; Kurakula et al. 2020 "Chapter 13 - Alginate-Based Hydrogel Systems for Drug Delivery in Wound Healing." However, pure alginate has poor mechanical properties, being a relatively rigid material and limiting its potential application to a different part of the body. For this reason, glycerol was introduced as a plasticizer in the sodium-alqinate film, thus obtaining a reduction in Young's modulus and tensile stress at maximum load and an increase in the elongation capacity of the films.

According to one embodiment, the dressing film can be obtained by adding to a solution comprising sodium alginate with a concentration of 4.6 to 5.2% w/v and PVP-I with a concentration of 1.3 to 1.9% w/v glycerol at a concentration of 1.3 to 2.3 w/w with respect to the total weight of sodium alginate and PVP-I.

This kind of dressing has the advantage of being naturally resorbed, thus avoiding the need for a detachment from the lesion and therefore the possibility of generating new lesions when replacing the dressing. After absorption, in fact, a new dressing can be easily applied simply by superimposing a new dressing film on the lesion and/or possibly what remains of the previous film.

According to one characteristic, the resorption of the dressing by the skin takes place particularly for a thickness of the aforementioned film less than 2000 µm, preferably less than 100, even more preferably from 75 to 95 µm.

As will be seen in more detail also from the experimental results, the combination of electrical stimulation according to a FREMS technology with the dressing using a film obtained from a solution of sodium alginate, povidone iodine and glycerol, allows to obtain a therapeutic effect on the treatment of ulcers, particularly of diabetic ulcers which is surprisingly and unexpectedly, considerably more effective, than the single treatments separated from each other or the simple sum of the therapeutic effects of the single actions of electro stimulation and pharmacological treatment.

This unexpected and surprising increase demonstrates a synergistic effect of the action of electrical stimulation and pharmacological action.

As regards the configuration of the biomedical device in the form of self-medication and therefore to be practically usable also directly by the patient, the present invention provides several advantageous embodiments.

These and other characteristics and advantages of the present invention will become clearer from the following description of some embodiments illustrated in the enclosed drawings wherein:
Fig. 1 illustrates a block diagram of an embodiment of the apparatus according to the invention.
Figures 2, 3 and 4 schematically show the three layers of an embodiment of the flexible support for a device according to the present invention.
Fig. 5 shows an embodiment of a flexible, adhesive support according to Figures 2 to 4 and which further comprises an extension for fixing a control device.
Fig. 6 shows an embodiment of a flexible, adhesive support for a control device and separate battery supply and communication interface with a remote device.
Figures 7 to 9 show different embodiments for the wearable application of a device according to the present invention.
Figure 10 shows photographs of the lesion area relating to a control group, a group treated with electrostimulation only according to a FREMS technology, a group treated with only PVP-I dressing and a group treated with the combination of electrostimulation according to the technology FREMS in combination with a PVP-I dressing according to the present invention and the graphic representation of the area of the lesion as a function of the days of treatment.

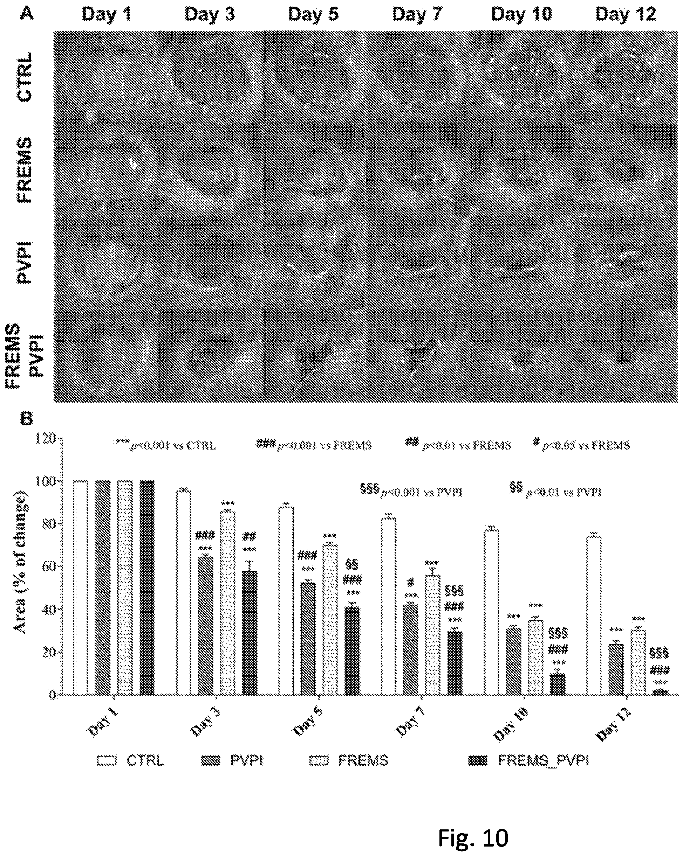

With reference to the more general embodiment of the present invention, the biomedical device for the treatment of ulcers, particularly diabetic ulcers, comprises a pair of electrodes for the transmission of electrostimulation impulses according to a FREMS technology said electrodes being meant to be applied in diametrically opposite positions with respect to the area presenting the lesion. As shown in Figure 10.

This figure compares the results obtained in the treatment of ulcers caused in diabetic mouse models.

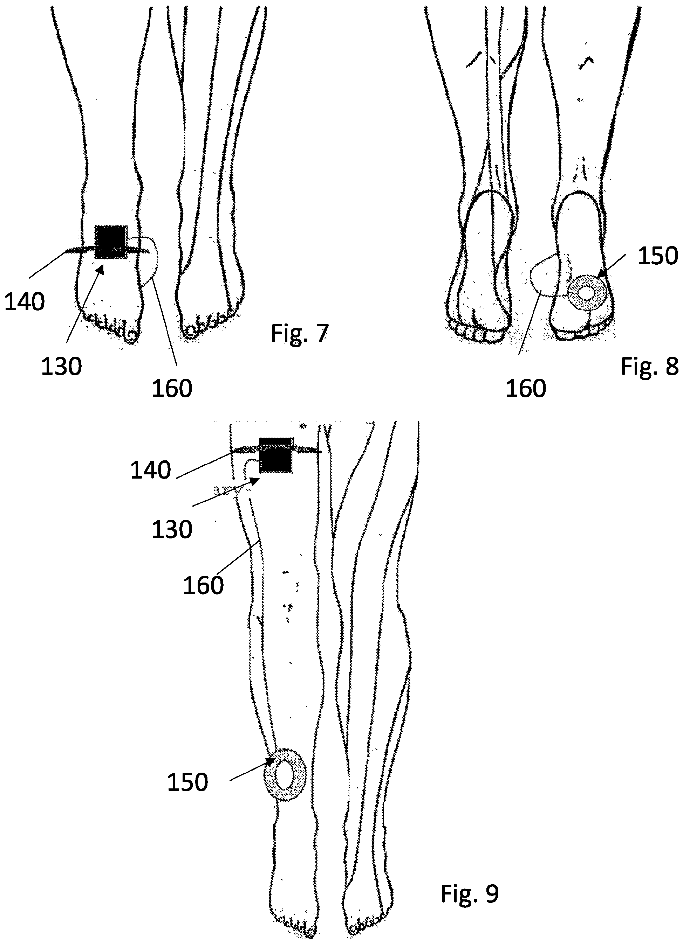

In these experiments, were considered untreated mice, mice treated only with the dressing consisting of a NaAlg/PVP-I film, mice treated only with an electrostimulation according to the FREMS technology and mice treated with a device according to the present invention which provides a pharmacological treatment with a dressing using a NaAlg/PVP-I film and simultaneously with an electrostimulation; the main results of the experiment are reported. As it can be seen, during the monitoring period of 12 days, in the untreated mice, called CTRL, the ulcerative lesion did not substantially undergo any healing due to the diabetic condition, showing at the end of the observation period 80% of the area of the wound still not healed. The application of FREMS technology showed a gradual wound healing during the observation period (12 days) and led to a recovery of 70% of the area initially affected by the wound. The FREMS technology was statistically better than the CTRL controls as also shown in the lower graph in Figure 9.

Mice treated with the PVP-I based dressing alone, identified with PVP-I, showed a slight difference in the progression of the wound healing process. In fact, as can be seen in Figure 9, a first major effect of the healing process was observed both with respect to controls and FREMS technology up to the first 7 days. After 12 days, mice treated with PVP-I based patches showed a residual wound area of 23%, which was statistically better than CTRL.

Finally, mice treated with both wound dressing and FREMS technology showed a surprising synergistic effect of the two therapies. In fact, from the very first days, the condition of the wound was statistically improved both in relation to the CTRL group and in relation to the group treated with electrostimulation only and the group treated with dressing only. It is important to note that after 5 days, the improvement in repair of the area affected by the lesion was statistically better than in mice treated with the PVP-I based patch alone. Finally, after 12 days, the mice treated with the combination of electro-stimulation according to the FREMS technology and the PVP-I dressing showed a complete recovery of the wounds.

According to these data, both NaAlg/PVP-I and FREMS alone were able to accelerate but not complete wound healing, while the combination of NaAlg/PVP-I and FREMS technology was able to heal the induced wound in 12 days, suggesting a positive synergistic effect of the two technologies.

In carrying out the experiment, the dressing film was prepared according to the following example:
A solution of Sodium Alginate was prepared at a concentration of 5% w/v, PVP-I (povidone iodine) at a concentration of 1.5% w/v in a final volume of 10 mL and glycerol at 1.6% w/w with respect to the total weight of alginate and PVP-I. 1 mL of this solution was placed on a 2 x 2 cm glass square. The glass with the solution was spin-coated at 180 rpm for 1.5 minutes. The resulting transparent films had an average thickness of 86 µm. After manufacturing, the films were punched into small discs with a diameter of 0.6 cm.

The combination of film and FREMS technology was evaluated in *in vivo* diabetic mouse models. The disease was induced by treating the animals with a dose of 50 mg/kg of Streptozotocin for 5 consecutive days. After a 4-week period, the blood glucose level of the mice was checked and the mice with blood glucose above 300 mg/dL were considered diabetic. Two experimental cycles were performed. In the first cycle, the speed of wound healing was studied by calculating the wound area at different times. The experiment lasted 12 days. In the second cycle, on the other hand, the level of inflammatory mediators was evaluated 5 days after the induction of the wound. In both groups, full-thickness excisional wounds were generated to begin the experiments.

In both experiments, immediately after the production of the wound, dressing films were applied to the wounds, said films being made according to the example described above. The next day, FREMS therapy began, and the mice were subjected to electrostimulation every day until the end of the experiments.

The electrostimulation program called "Traumatic Ulcers" (described in detail in documents IT 1341343, WO2004067087A1, WO2004/084988 and particularly EP1305080) lasting 30 minutes. The voltage was reduced considering the small body size of the mice compared to human applications described in the documents listed above. The voltage was then reduced to 15 V. The pulse sequences according to FREMS technology were applied by means of two adhesive electrodes directly on the shaved backs of the mice, one of the electrodes was positively charged and the other negatively. These electrodes were replaced with new ones each time after use to ensure full contact between the electrodes and the skin of the mice. During the electrostimulation the two electrodes were applied at a distance of 1 cm from the wound and in opposition to each other. Every day the two electrodes were applied in reverse, i.e., their polarity has been inverted.

During the experiments, the patch was applied immediately after the wound was generated. The next day, FREMS treatment began in the aforementioned "Traumatic Ulcers" mode, limiting the voltage to 15V.

Disturbances in blood flow and persistence of inflammatory conditions are the main reasons for unresolved diabetic ulcers. Therefore, the measurement of the level of inflammatory mediators was performed after the application of the two combined technologies.

After 5 days of injury induced damage in the *in vivo* diabetic mouse model, TNF-α levels were higher in the CTRL control group, while the values in the group of mice treated with FREMS electrostimulation alone, or with the PVP-I dressing alone and in the group of mice treated with the combination of FREMS electrostimulation and PVP-I dressing were statistically lower. Furthermore, in the group treated with the PVP-I dressing only and in the group treated with the combination of FREMS electrostimulation and PVP-I dressing according to the present invention, these values were also statistically lower than the values of the group treated only with electrostimulation according to FREMS technology.

Also the levels of IL-6 in the group of mice treated only with electrical stimulation according to FREMS technology, in the group of mice treated with only the PVP-I dressing and in the group of mice treated with the combination of FREMS electrostimulation and PVP-I dressing were statically reduced compared to the CTRL control group. However, in this case, treatment with the combination of FREMS electrostimulation and PVP-I medication produced the lowest levels of IL-6, being statistically lower even than the values for the group of mice treated with only electrostimulation with FREMS technology and in the group treated with only PVP-I dressing.

A similar trend was also observed for IL-1β values with treatment with the combination of FREMS electrostimulation and PVP-I medication, showing the best results in reducing the level of this inflammatory mediator.

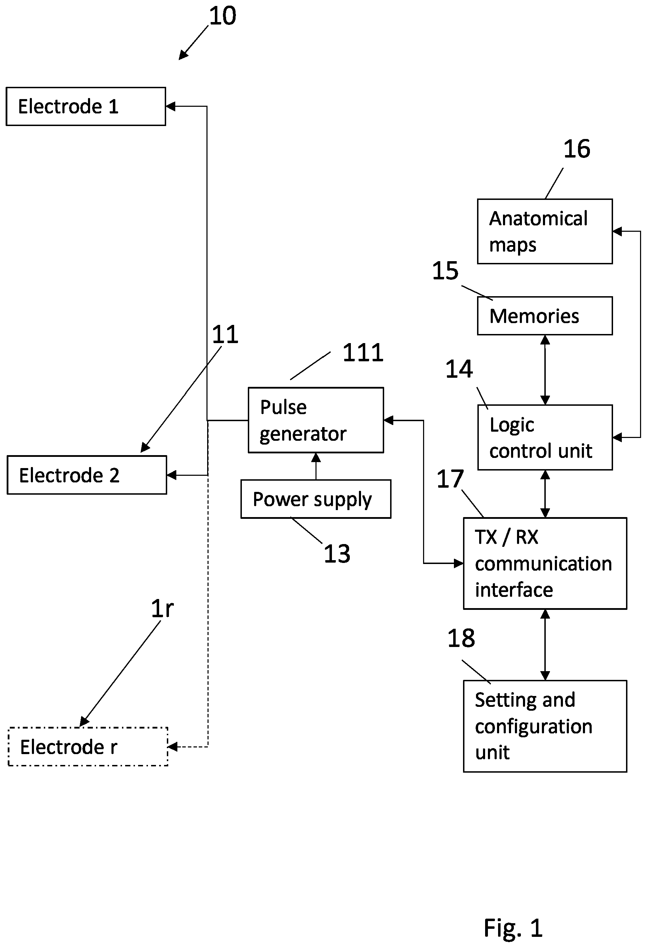

Figures 1 to 8 show different embodiments of a biomedical device for the treatment of ulcers, particularly diabetic ulcers according to the more general concept described above.

In the following description, a flexible support element means a sheet or a flattened element to which are applied or wherein are incorporated by lamination of two or more layers or by drowning, constructive parts such as electrodes, conductors for the transmission of signals or pockets for drugs or for electronic circuits.

Furthermore, the flexible support elements described are intended to be provided with a continuous layer of adhesive or with zones for removable fixing to the skin of the human body.

Figure 1 illustrates the block diagram of an electrostimulation apparatus, which according to the present invention is made in the form of a wearable apparatus.

The apparatus comprises one or more flexible supports indicated with 10, 11, 1r. Each flexible support bears stimulation electrodes 1, 2, r, the variable r being indicative of any natural number, since the individual supports 10, 11, 1r may be provided with an identical or a different number of electrodes. In the embodiment of figure 1, the support 1r bears an electrode r and is illustrated with broken lines in order to show a possible embodiment which provides for more than two electrodes as provided in the previous experiment and in the preferred embodiment of the present invention.

However, it is possible to optionally provide more than two electrodes, each with its separate flexible element for supporting and adhering to the patient's body in a prefixed area and with a prefixed relative position with respect to the other electrodes.

A generator 111, with an associated power supply 13 has an output channel for each electrode 1, 2, r through which a sequence of electrical stimulation pulses is fed to the corresponding electrode. The pulse generator 111 operates under the control of a logic control unit 14.

This logic control unit is configured to provide the generators with instructions relating to the pulses to be generated in relation to the configuration parameters of said pulses. These parameters are related to one or more of the following quantities: amplitudes, intensity, power, frequency, duration, polarity, waveform and to generate a combination of a temporal succession of different pulses for one or more of the aforementioned parameters as well as to synchronize among them the pulses of the sequences supplied to the electrodes 1, 2, r for their delivery through the skin to the patient.

In the embodiment of Figure 1, a single logic control unit is provided to control the generator 111 in a synchronized way, but as will appear below, in this case it is a possible embodiment, being possible to provide alternative architectures which provide for two or more control units dedicated to a single generator or to subgroups of generators and which operate in a synchronized way with each other, automatically negotiating the control timing sequences of the generators.

The logic control unit 14 can be made either in the form of dedicated hardware wherein the logic control of the generators is steadily integrated according to one or more options that are selectable but substantially fixed.

Another embodiment, on the other hand, provides that the logic control unit is constituted by generic hardware comprising a processor and peripherals and that the logic control unit 14 executes a control software which is stored in a memory 15. In relation to the specific application of the stimulating apparatus, the memory 15 can contain databases of different settings corresponding to different types of treatment, both as regards the anatomical region and as regards the effects to which the treatment is aimed.

According to a non-limiting embodiment, a memory area 16 can be provided which is dedicated to anatomical maps of positioning of the electrode(s) and/or of the flexible supports 10, 11, 1r in relation to the different conditions of the lesions to be treated and/or the anatomical areas wherein they are present.

Always according to a further embodiment which can be provided in any combination with one or more of the embodiments and variants previously described, it is possible to provide a communication interface 17 which allows the control unit to deliver the command signals to the generator 111 and to a setting and configuration unit 18 or to a man-machine interface unit which allows to perform manual maintenance, setting and configuration operations as well as to upgrade the program executed by the logic control unit 14 and/or the databases of the treatment protocols and/or of the anatomical maps of electrode positioning and possibly also of performing diagnostic activities of the units of the apparatus. The communication can take place both by means of cables and by radio, i.e. wireless.

The choice of the communication mode and protocols among those currently known by the skilled in the art is a pure opportunity choice dictated by the required bandwidth, the required signal power, the energy resources provided by the power supply and depends on the kind of architecture of the apparatus and the kind of treatment to be performed.

The setting and configuration unit 18, or the interfacing unit, can consist of a remote unit, such as a mobile device made available to the user. This can also be a mobile device of the kind currently known wherein is installed an application which when executed configures the mobile device to perform the functions of the interface unit 18 of the apparatus.

Particularly advantageous examples of this kind of mobile unit can be devices such as smartphones, phablets, tablets or similar devices.

A preferred but non-limiting embodiment provides that the generator 11, and all or at least some of the units described above 13, 14, 15, 16, 17 and 18, when present, are integrated in a single control device.

In this case, the control unit that integrates all said units or a part of them can consist of a dedicated hardware that incorporates in the hardware itself all the foreseen functions, or said device consists of a generic processing unit which executes a program wherein the instructions are encoded to make such hardware capable of performing the functions required for the execution of the electrostimulation according to the FREMS protocols envisaged in the present invention.

The generator and particularly the control device wherein said generator can be integrated together with one or more of the other units of figure 1, can be produced as a wearable device, thanks to a wearable support that can be produced according to different embodiments.

With regard to flexible supports, an embodiment can consist of a support film, for instance of canvas or plastic material, to one of the faces of which a layer of adhesive material is applied. The electrode can consist of a sheet of conductive material cut with a prefixed shape and size and which is applied to the adhesive material. Each pole of each electrode is made from a piece in the form of a sheet of electrically conductive material and a power supply conductor is connected to each pole, for instance in the form of a band of conductive material.

The sheet-shaped pieces of conductive material can be attached to the adhesive layer. The conductors connecting the electrodes to the outputs of the channels of the generator or of the control device can also consist of tracks or bands of a sheet of electrically conductive material. As will be described later.

The electrodes and the conductive tracks or bands are further covered to be electrically insulated towards the outside by a band of plastic material, for instance a double-sided adhesive band, which on one side overlaps the conductors or conductive bands adhering against them and against the adhesive layer of the flexible support, while the other side of the double-sided tape restores the continuity of the adhesive layer in correspondence with the path of the conductors.

Several other construction forms are possible, for instance it is possible to provide that the individual layers are laminated on each other or that the conducting elements are fixed in a plastic matrix by molding. A further embodiment can provide for the production of the poles of the electrodes and/or of the conductive tracks by applying them using electrically conductive liquids which are sprayed to form the poles of the electrodes and/or the conductive tracks of the same.

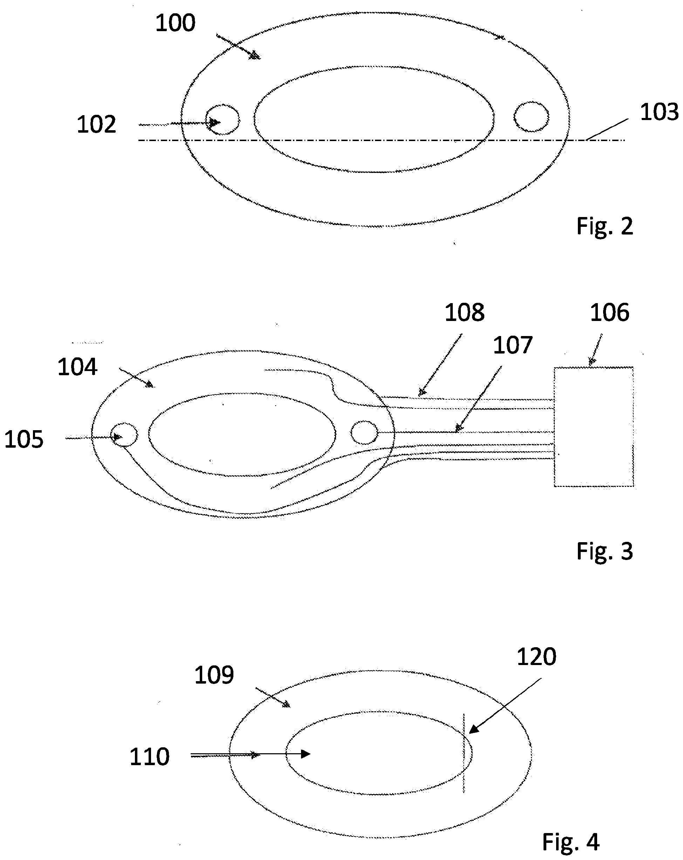

According to an embodiment illustrated in Figures 2 to 4, the flexible support for at least two electrodes provided in the preferred embodiment of the present invention is made in the form of a frame that delimits a central window. The frame can be essentially annular in shape which can have any shape such as circular, elliptical, oval, or even polygonal.

On the annular shape it is possible to distribute one or more electrodes 102, in this case two electrodes in diametrically opposite positions.

The flexible element in the shape of an annular frame can bear the connecting conductors of the same integrated inside it as described above, or in the form of electric cables fixed to the external visible surface of said flexible element at least for part of their length to the corresponding generator channel.

The central window is intended to accommodate the lesion or ulcer to be treated, being in this case the dimensions of the frame and/or its shape made so as to form a window of sufficient size to accommodate the lesion and at the same time to determine a position of each electrode relative to the edges of the lesion corresponding to a prefixed optimal distance.

According to one embodiment, this optimal distance is of the order of magnitude of a few centimeters, preferably at least one centimeter. Furthermore, the conformation of the annular frame and the position of the electrodes thereon is such as to make evident the relative positioning of the two electrodes on diametrically opposite sides with respect to the center of the lesion.

A preferred form which allows easy recognition of the disposition of the electrodes with respect to the lesion is the oblong, particularly elliptical, embodiment shown in the figures, however this embodiment, even if preferred, must not be considered limiting.

As far as the dressing film is concerned, this is made with shape and dimensions substantially corresponding at most to the shape and dimensions of the lesion positioning window delimited by the frame.

The dressing film made according to one or more of the previously described embodiments is provided as a separate element with respect to the flexible element that bears the electrodes and must be applied in a subsequent step to the application of the flexible element that bears the electrodes.

According to a still further advantageous feature, the flexible support can be made as a multilayer as indicated in Figures 2 to 4. In this case, a first layer consists of the layer 100 in contact with the skin. This layer is made of biocompatible material, such as preferably polyurethane material.

Said material can advantageously be further permeable to gases and is provided with a layer of adhesive on the part intended to come into contact with the skin.

According to an embodiment, the adhesive is of medium grade.

Still according to a further embodiment, said layer is of transparent material and/or of thin thickness and in any case such as to be sufficiently plastic to allow deformation both in application to the skin and in movement.

The annular element 100 has special housing areas for adhesion of two electrodes distributed at the two ends of the major axis of the elliptical shape of said ring 100.

These housing areas consist of Ag-Cl gel and generate contact between the electrode carried by the ring 104 and the patient's skin.

The second layer 104 of the flexible support is shown in Figure 3. The second layer has an annular shape essentially congruent to that of the first layer 100 and adheres to said first layer by chemical and physical adhesion. The second layer constitutes a non-disposable system of more rigid material, preferably of biocompatible polyurethane.

According to a preferred embodiment, the second layer is also of gas permeable material and includes the circuit part indicated with 107, 108.

Preferably said circuit part is made as a flat cable or in the form of electrically conductive tracks as described above.

The ring 104 of the second layer bears in a position coinciding with the areas 102 of the first layer a corresponding electrode 105 which adheres in contact with the gel of said areas 102.

A control device 106 connects to the conductors 107, 108 and drives the electrodes. The control device 106 can be made according to one or more of the embodiments described with reference to Figure 1 and can be mounted steadily or in a removable way, for instance in an openable and closable pocket of an adhesive flexible element which has a contact surface with the skin. This flexible support element of the control device 106 is also made of biocompatible material, preferably of biocompatible polyurethane.

According to a preferred embodiment, said material is also adhesive on the surface in contact with the skin.

In combination with the adhesive layer, it is possible to provide a mechanical fixing by tightening thanks to an elastic band, not adhesive such as a band provided with a velcro closure, snap buttons or the like. This band indicated with 140 in figures 7 to 9 has the function of safety fixing of the control unit 106.

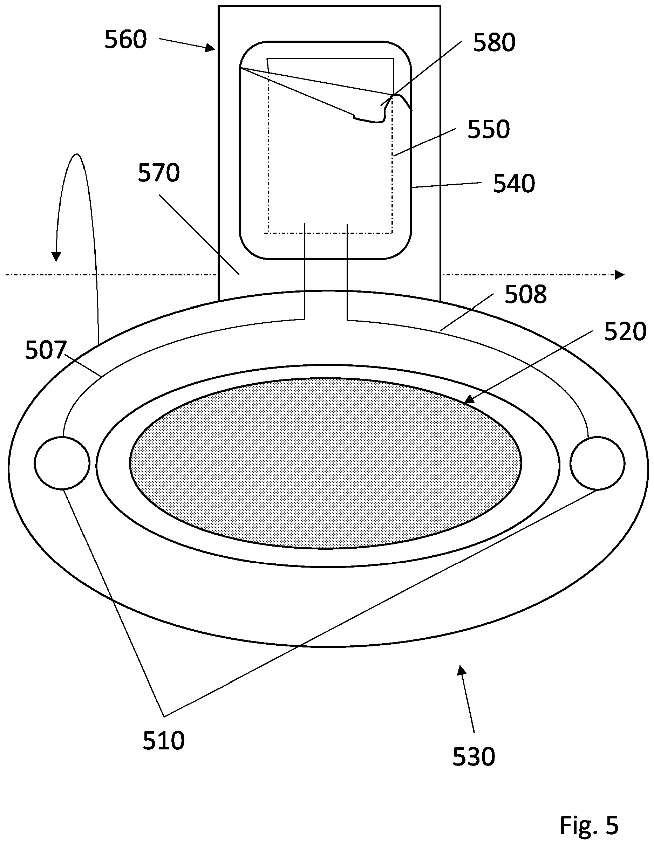

In the embodiment wherein the connection between the electrodes and the corresponding output of the control device is of the conductive band or track kind steadily integrated in the flexible element, the control device 106 is steadily fixed to an extension 560 of the frame 530 as shown in figure 5. In this case, in the "not in use" condition, said extension 560 can be foldable against the frame element 530 and can be turned outwards in the condition of application at the moment of use.

The control device indicated with 550 is inserted in a housing pocket 540.

Inside the elliptical element 530, the dressing film applied above the lesion is placed in the window which is centered with respect to said frame element 530 and also provided inside the window.

In a possible embodiment the control device is steadily housed in position between two layers 570 and 580. The conductors 507, 508 are steadily connected to the electrodes 510 and to the corresponding outputs of the control device 550.

In another embodiment, the extension 560 of the flexible element forms an openable pocket so that the control device 550 is removable. In this case, the two conductors 507, 508 can end in a contact base which cooperates with a coupling terminal provided on the control device 550, in such a way that by inserting the same in the pocket, the connection with the control device is automatically generated and extracting the control device from the pocket said contact base is separated from the coupling terminal, interrupting the contact with the electrodes and releasing the control device itself relating to its extraction from the pocket.

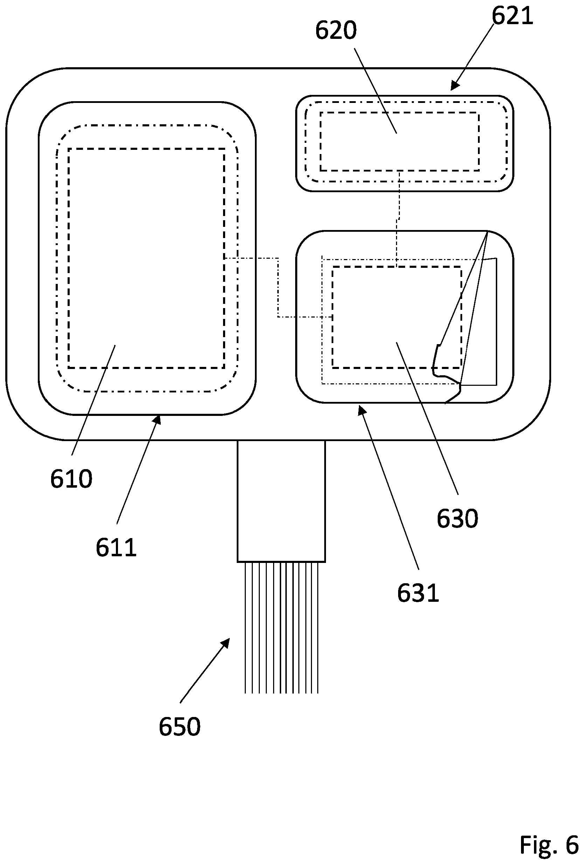

Figure 6 shows a further embodiment wherein the control device does not integrate all the units referred to in the previous description according to the embodiment of Figure 1, but these are divided into three separate units, each supported together on a flexible element that can be separated from the flexible element supporting the electrodes or connected to the latter as in the example of Figure 5.

In this embodiment is provided a communication unit in transmission and reception 620 and a power supply battery 630 which are separated from the control device 610 which integrates all the other units such as the generator, the control unit, the memories referred to in Example of Figure 1. The control device 610, the communication unit 620, the battery 630 are housed in a corresponding pocket 611, 621 and 631 respectively.

As illustrated, at least the pocket 631 of the supply battery 630 can be opened to allow it to be replaced with a charged battery.

Alternatively, one or more of the additional pockets can be opened or the battery pocket cannot be opened, but the battery is of the rechargeable kind and has an externally accessible connection socket with a recharging power supply or recharging can be performed using wireless chargers.

In one embodiment, this battery socket can be accessed through a window (not shown) in the wall of pocket 631 which can be openable and closable or always open.

The pockets 611, 621 and 631 are provided for instance on a flexible support element 600, for instance of the adhesive kind such as those carrying the electrodes.

Figure 6 illustrates a variant wherein the connection of the control device 610 with the electrodes is made by means of independent electrical conductors for each electrode and which are indicated with 650.

Said conductors extend up to the connection connectors of the generators present on each support element for the electrodes and bear corresponding connection pins.

The connection among control device 610, communication unit 620 and battery 630 can take place through conductive tracks or cables, particularly flat cables, integrated in the flexible element 600.

With reference to Figure 4, here is illustrated a further variant of the embodiment, wherein in addition to the first and second layers of Figures 2 and 3, said embodiment has a third layer indicated as 109 in Figure 4.

According to an embodiment, this third layer is of disposable material and has the function of covering the second layer shown in Figure 3 towards the outside.

According to a further characteristic, the third layer can further cover also the central area of the annular shape of the two underlying layers, extending also along said central part.

Advantageously, the central part is retained by the thickness of the two underlying layers lifted up with respect to the skin and in the part coinciding with the window is free of adhesive so as not to adhere to the part of the lesion or to the dressing film.

According to yet another embodiment, the third layer 109 can act as an element for closing and opening said central zone of the annular shape of the underlying layers. Said central window indicated with 110 being intended to receive the lesion to be treated and the dressing film.

In an embodiment, on one side of said central zone, the third layer is provided or is shaped in such a way as to form a hinge as indicated schematically by line 120. Said hinge is advantageously elastic and has the function of allowing repeated opening and closing actions, for instance to allow the replacement of the dressing film or the like.

According to a preferred embodiment, the material of the third layer has a greater rigidity than the first layer and is in any case made of biocompatible and/or gas-permeable material.

According to another embodiment, it is possible to provide that inside the window delimited by the frame can be provided a removable layer of biocompatible polyurethane sponge which has a prefixed thickness and has a prefixed capacity to absorb liquids, particularly for the removal of the exudate by periodic replacement.

With reference to another embodiment that can be derived from that according to figures 2 to 4, in place of a frame closed on itself, it is possible to provide a flexible element which is provided with two branches connected to each other and which form a concave area intended to accommodate the wound and the dressing film.

The electrodes are provided at the terminal ends of the two branches which are curved or slanted with respect to each other and have a length such that said ends that bear the electrodes are diametrically opposite with respect to the central bisector axis of the concavity.

With reference to the specific embodiment of figures 2 to 4, it is possible to provide that the flexible element ends at the level of the section line indicated with 103.

Figures 7 to 9 show two examples of application of the apparatus described in figures 2 to 4.

130 indicates the control unit and 140 indicates the fastening by tightening element. 150 indicates the flexible support composed of the three layers of the example of figures 2 to 4.

160 indicates the connection conductors of the control unit 130 to the electrodes of the flexible support according to figures 2 to 4.

The lesion is placed inside the window of the flexible element in the shape of a frame and the dressing film is applied to it.

## Claims

1. A biomedical device for the therapeutic treatment of ulcers or lesions, particularly diabetic ulcers, said device consisting of a kit comprising in combination an electro-stimulator device and a dressing comprising a pharmaceutical composition and wherein the electro-stimulating device and the said dressing are applied simultaneously for treating an ulcer and which device comprises:
- at least an electrical pulses generator (111), said electrical pulses being organized in sequences of electrical pulses having prefixed values of typical parameters, said typical parameters comprising one or more of the following parameters: amplitude, duration, frequency and/or waveform of said pulses and said parameters being configured according to the technology called FREMS acronym for Frequency Rhythmic Electric Modulation;
- said generator (111) comprising at least two separate stimulation channels, through which said sequences are delivered to body areas of an organism independently for each channel;
- at least two stimulation electrodes (1, 2, 102, 105), each of which is connected or connectable to a respective stimulation channel and each of which is intended to be removably applied to one of two different prefixed body zones of an organism;
- each electrode (1, 2, 102, 105) being applied externally to the patient's skin in correspondence with said prefixed area and with a prefixed position relationship with respect to the position of the remaining electrode or electrodes;
- a control unit (14; 130) of said at least one electric pulse generator (111) communicating with said electric pulse generator;
- a command and/or data entry interface for setting and/or displaying setting data and/or setting settings of said one or more generator (111);
- said dressing consisting of a film comprising a solution of povidone iodine (PVP-I) and sodium alginate (NaAlq) and intended to be applied while treating the ulcer;
and wherein
- the at least two electrodes (1, 2, 102, 105) are intended to be arranged on diametrically opposite sides of the lesion at a prefixed distance therefrom.

2. The device according to claim 1, wherein the distance between each the position of each electrode( 1, 2, 102, 105) when applied to the patient's body and the edge of the lesion or of the ulcer facing the corresponding electrode is of the order of at least a few centimeters, particularly of at least 1cm.

3. The device according to claims 1 or 2 wherein the dressing consists of a solution of Sodium Alginate, PVP-I and glycerol.

4. The device according to one or more of the previous claims wherein the dressing consists of a film obtained by adding to a solution comprising sodium alginate with a concentration of 4.6 to 5.2% w/v and PVP-I with a concentration of 1.3 to 1.9% w/v and glycerol at a concentration of 1.3 to 2.3 w/w with respect to the total weight of sodium alginate and PVP-I.

5. The device according to one or more of the previous claims, wherein said at least two electrodes (1, 2, 102, 105) are each beared by an independent flexible self-adhesive support element (10, 11; 150; 600), which can be fixed in a prefixed position in relation to the anatomy of the human body.

6. The device according to one or more of the previous claims 1 to 4, wherein said at least two electrodes (1, 2, 102, 105) are beared by a single flexible, self-adhesive support element (150), which can be fixed in a prefixed position in relation to the anatomy of the human body, said flexible element having at least two branches which delimit an area at least of concave shape for housing the lesion, while said electrodes are each provided on one end of the respective branch, said concave area being configured to house a dressing film.

7. The device according to one or more of claims 5 or 6, wherein said flexible element is shaped like a frame closed on itself which has a free central area intended to accommodate the lesion, said at least two electrodes (1, 2, 102, 105) being provided on said frame in two points of the same diametrically opposite each other with respect to the center of said frame, while the dressing consists of a film of such shape and size as to be at least inscribable inside said frame.

8. The device according to one or more of the previous claims 5 to 7 wherein a closure layer is provided which overlaps, at least partially, the frame and/or the support branches of the electrodes (1, 2, 102, 105) and also the concave area or the central area, adhering only to one or to the flexible supporting elements (150) of the electrodes and therefore to the frame and/or the two supporting branches of the electrodes and not having an adhesive action in correspondence with the lesion and the dressing film.

9. The device according to one or more of the previous claims, wherein a series of flexible elements (10, 11; 150; 600 for supporting the electrodes (1, 2, 102, 105) and/or dressing film with shapes and/or sizes intended for lesions having different shapes and/or sizes is provided.

10. The device according to one or more of the previous claims wherein the aforementioned generator (111), the aforementioned control unit (14; 130) and the aforementioned command and/or display interface (17, 18) and the power source (13) of these components are integrated in a single control device (106; 550; 610), said device being connected or connectable by means of electric cables (107, 108) to the aforementioned electrodes (1, 2, 102, 105) and said connection being able to be fixed, or the connection can be made by means of coupling terminals provided at the end of the connection cable with the corresponding electrode, which are connected in a separable way to corresponding connection terminals provided with said corresponding electrode.

11. The device according to one or more of the previous claims wherein the control device (106) is provided in combination with a wearable element for removable fastening of said control device to an anatomical region of the patient.

12. The device according to claim 11, wherein said wearable elements are provided with closable and openable housing pockets for said control device (106).

13. The device according to claims 11 or 12, wherein said wearable elements, in the form of flexible elements, consist of one or more external side extensions of a branch of the at least two branches of the flexible support element of the electrodes (1, 2, 102, 105) or of the flexible support element in the form of a frame.

14. The device according to one or more of the previous claims 5-9 or 13, wherein conductors connecting the electrodes to the corresponding channel of the generator (111) are integrated into the structure of the flexible element.

15. The device according to one or more of the previous claims 5-9 or 13-14, wherein a support element of the control device (106) has a terminal for connecting conductors connecting the electrodes to the control device which consists of a connection base steadily fixed to said flexible element.

16. The device according to one or more of the previous claims 5-9 or 13-15, wherein said flexible element being shaped so as to delimit a concave zone and/or a window surrounded by a frame closed on itself and comprises at least one element of a film or a plurality of elements of said dressing film having a shape substantially congruent with the zone delimited by the flexible support element, being the said control device (106) removably applicable to said flexible element and being optionally comprised a cover sheet of the concave area and/or said window which can be removably applied to the face opposite the application surface of the flexible element and which overlaps at least partially to said concave area and/or to said window and/or to said control device.

17. A kit for the therapeutic treatment of ulcers wherein the combination of flexible support element for two electrodes (1, 2, 102, 105) and support of at least one control device (106) and at least one dressing film comprises at least a flexible element and a device according to one or more of claims 1 to 16.

## Patentansprüche

1. Biomedizinische Vorrichtung zur therapeutischen Behandlung von Geschwüren oder Läsionen, insbesondere diabetischen Geschwüren, wobei die Vorrichtung aus einem Kit besteht, das in Kombination eine Elektrostimulatorvorrichtung und einen eine pharmazeutische Zusammensetzung umfassenden Verband umfasst, und wobei die Elektrostimulatorvorrichtung und der Verband gleichzeitig zur Behandlung eines Geschwürs angewendet werden, und wobei die Vorrichtung umfasst:
- mindestens einen Generator (111) für elektrische Impulse, wobei die elektrischen Impulse in Sequenzen von elektrischen, mit vorher festgelegten Werten typischer Parameter versehenen Impulsen organisiert sind, wobei die typischen Parameter einen oder mehrere der folgenden Parameter umfassen: Amplitude, Dauer, Frequenz und/oder Wellenform der Impulse, und wobei die Parameter gemäß der als FREMS bezeichneten Technologie - Akronym für Frequency Rhythmic Electric Modulation - konfiguriert sind;
- wobei der Generator (111) mindestens zwei getrennte Stimulationskanäle umfasst, durch die die Sequenzen unabhängig für jeden Kanal an Körperbereiche eines Organismus abgegeben werden;
- mindestens zwei Stimulationselektroden (1, 2, 102, 105), von denen jede mit einem entsprechenden Stimulationskanal verbunden oder verbindbar ist und von denen jede zum abnehmbaren Anlegen an eine von zwei verschiedenen vorher festgelegten Körperbereichen eines Organismus eingerichtet ist;
- wobei jede Elektrode (1, 2, 102, 105) außen auf der Haut des Patienten im Bereich der vorher festgelegten Bereiche und mit einer vorher festgelegten Lagebeziehung zur Lage der übrigen Elektrode oder Elektroden angeordnet wird;
- eine Steuereinheit (14; 130) des mindestens einen elektrischen Impulsgenerators (111), die mit dem elektrischen Impulsgenerator in Verbindung steht;
- eine Befehls- und/oder Dateneingabeschnittstelle zum Einstellen und/oder Anzeigen von Einstelldaten und/oder zum Setzen von Einstellungen des einen oder der mehreren Generatoren (111);
- wobei der Verband aus einer Folie besteht, die eine Lösung von Povidon-Iod (PVP- I) und Natriumalginat (NaAlg) umfasst und zum Auftragen während der Behandlung des Geschwürs ausgebildet ist;
und wobei
- die mindestens zwei Elektroden (1, 2, 102, 105) dazu ausgelegt sind, auf diametral gegenüberliegenden Seiten der Läsion in einem vorbestimmten Abstand davon angeordnet zu werden.

2. Vorrichtung nach Anspruch 1, wobei der Abstand zwischen der Position jeder am Körper des Patienten angelegten Elektrode (1, 2, 102, 105) und dem der entsprechenden Elektrode zugewandten Rand der Läsion oder des Geschwürs in der Größenordnung von mindestens einigen Zentimetern liegt, insbesondere mindestens 1 cm beträgt.

3. Vorrichtung nach Anspruch 1 oder 2, wobei der Verband aus einer Lösung von Natriumalginat, PVP-I und Glycerin besteht.

4. Vorrichtung nach einem oder mehreren der vorhergehenden Ansprüche, wobei der Verband aus einem Film besteht, der erhalten wird, indem zu einer Lösung, die Natriumalginat mit einer Konzentration von 4,6 bis 5,2 % Gew./Vol. und PVP-I mit einer Konzentration von 1,3 bis 1,9 % Gew./Vol. enthält, Glycerin in einer Konzentration von 1,3 bis 2,3 Gew./Gew., bezogen auf das Gesamtgewicht von Natriumalginat und PVP-I, zugegeben wird.

5. Vorrichtung nach einem oder mehreren der vorhergehenden Ansprüche, wobei die mindestens zwei Elektroden (1, 2, 102, 105) jeweils von einem unabhängigen, flexiblen, selbstklebenden Trägerelement (10, 11; 150; 600) getragen werden, das in einer vorbestimmten Position in Bezug auf die Anatomie des menschlichen Körpers fixiert werden kann.

6. Vorrichtung nach einem oder mehreren der vorhergehenden Ansprüche 1 bis 4, wobei die mindestens zwei Elektroden (1, 2, 102, 105) von einem einzigen flexiblen, selbstklebenden Trägerelement (150) getragen werden, das in einer vorgegebenen Position in Bezug auf die Anatomie des menschlichen Körpers fixiert werden kann, wobei das flexible Element mindestens zwei Schenkel aufweist, die einen Bereich mit wenigstens einer konkaven Form zur Aufnahme der Läsion begrenzen, während die Elektroden jeweils an einem Ende des jeweiligen Schenkels vorgesehen sind, wobei der konkave Bereich zur Aufnahme einer Verbandfolie ausgebildet ist.

7. Vorrichtung nach einem oder mehreren der Ansprüche 5 oder 6, bei der das flexible Element die Form eines in sich geschlossenen Rahmens hat, der einen freien zentralen Bereich zur Aufnahme der Läsion aufweist, wobei die mindestens zwei Elektroden (1, 2, 102, 105) auf dem Rahmen an zwei einander diametral gegenüberliegenden Punkten in Bezug auf die Mitte des Rahmens vorgesehen sind, während der Verband aus einer Folie mit einer solchen Form und Größe besteht, dass sie zumindest im Inneren des Rahmens eingeschrieben werden kann.

8. Vorrichtung nach einem oder mehreren der vorhergehenden Ansprüche 5 bis 7, wobei eine Schließschicht vorgesehen ist, die den Rahmen und/oder die Stützschenkel der Elektroden (1, 2, 102, 105) und auch den konkaven Bereich oder den zentralen Bereich zumindest teilweise überlappt, wobei sie nur an einem oder an den flexiblen Stützelementen (150) der Elektroden und somit an dem Rahmen und/oder den beiden Stützschenkeln der Elektroden anliegt und keine Klebewirkung im Bereich der Läsion und der Verbandfolie hat.

9. Vorrichtung nach einem oder mehreren der vorhergehenden Ansprüche, wobei eine Reihe von flexiblen Elementen (10, 11; 150; 600) vorgesehen ist, die die Elektroden (1, 2, 102, 105) und/oder die Verbandfolie mit Formen und/oder Größen tragen, welche für Läsionen mit unterschiedlichen Formen und/oder Größen bestimmt sind.

10. Vorrichtung nach einem oder mehreren der vorhergehenden Ansprüche, wobei der besagte Generator (111), die besagte Steuereinheit (14; 130) und die besagte Befehls- und/oder Anzeigeschnittstelle (17, 18) sowie die Energiequelle (13) dieser Komponenten in einem einzigen Steuergerät (106; 550; 610) integriert sind, wobei die Vorrichtung mittels elektrischer Kabel (107, 108) mit den besagten Elektroden (1, 2, 102, 105) verbunden oder verbindbar ist und die besagte Verbindung fixiert werden kann, oder die Verbindung mittels Kupplungsanschlüsse hergestellt werden kann, die am Ende des Verbindungskabels mit der entsprechenden Elektrode vorgesehen sind, die mit entsprechenden, der besagten entsprechenden Elektrode zugeordneten Verbindungsanschlüssen lösbar verbunden sind.

11. Vorrichtung nach einem oder mehreren der vorhergehenden Ansprüche, wobei die Steuervorrichtung (106) in Kombination mit einem tragbaren Element zur lösbaren Befestigung der Steuervorrichtung an einem anatomischen Bereich des Patienten vorgesehen ist.

12. Vorrichtung nach Anspruch 11, wobei die tragbaren Elemente mit verschließbaren und öffenbaren Aufnahmetaschen für die Steuervorrichtung (106) versehen sind.

13. Vorrichtung nach Anspruch 11 oder 12, wobei die als flexible Elemente ausgebildeten tragbaren Elemente aus einer oder mehreren äußeren Seitenverlängerungen eines Schenkels der mindestens zwei Schenkel des flexiblen Trägerelements der Elektroden (1, 2, 102, 105) oder des flexiblen Trägerelements in Form eines Rahmens bestehen.

14. Vorrichtung nach einem oder mehreren der vorhergehenden Ansprüche 5-9 oder 13, wobei in die Struktur des flexiblen Elements Leiter integriert sind, die die Elektroden mit dem entsprechenden Kanal des Generators (111) verbinden.

15. Vorrichtung nach einem oder mehreren der vorhergehenden Ansprüche 5-9 oder 13-14, wobei ein Trägerelement der Steuervorrichtung (106) einen Anschluss aufweist, der die Leiter, die die Elektroden mit der Steuervorrichtung verbinden, mit dem flexiblen Element verbindet, und der aus einem fest mit dem flexiblen Element verbundenen Verbindungssockel besteht.

16. Vorrichtung nach einem oder mehreren der vorhergehenden Ansprüche 5-9 oder 13-15, wobei das flexible Element so geformt ist, dass es einen konkaven Bereich und/oder ein Fenster begrenzt, das von einem in sich geschlossenen Rahmen umgeben ist, und mindestens ein Element einer Folie oder eine Vielzahl von Elementen der Verbandfolie umfasst, die eine mit dem durch das flexible Trägerelement begrenzten Bereich im Wesentlichen kongruente Form aufweist, wobei die Steuervorrichtung (106) an dem flexiblen Element lösbar anbringbar ist und optional eine Abdeckfolie des konkaven Bereichs und/oder des Fensters vorgesehen ist, die an der der Anbringungsfläche des flexiblen Elements gegenüberliegenden Seite lösbar angebracht werden kann und die zumindest teilweise den konkaven Bereich und/oder das Fenster und/oder die Steuervorrichtung überlappt.

17. Kit zur therapeutischen Behandlung von Geschwüren, wobei die Kombination aus flexiblem Trägerelement für zwei Elektroden (1, 2, 102, 105) und Träger von mindestens einer Steuervorrichtung (106) und mindestens einer Verbandfolie mindestens ein flexibles Element und eine Vorrichtung nach einem oder mehreren der Ansprüche 1 bis 16 umfasst.

## Revendications

1. Un dispositif biomédical pour le traitement thérapeutique d'ulcères ou de lésions, notamment d'ulcères diabétiques, ledit dispositif étant constitué d'un kit comprenant en combinaison un dispositif électro-stimulateur et un pansement comprenant une composition pharmaceutique et dans lequel le dispositif électro-stimulateur et le pansement sont appliqués simultanément pour traiter un ulcère et lequel dispositif comprend:
- au moins un générateur d'impulsions électriques (111), lesdites impulsions électriques étant organisées en séquences d'impulsions électriques ayant des valeurs prédéfinies de paramètres typiques, lesdits paramètres typiques comprenant un ou plusieurs des paramètres suivants: amplitude, durée, fréquence et/ou forme d'onde desdites impulsions et lesdits paramètres étant configurés selon la technologie appelée FREMS, acronyme de Frequency Rhythmic Electric Modulation (Modulation Électrique Rythmique en Fréquence);
- ledit générateur (111) comprenant au moins deux canaux de stimulation distincts à travers lesquels lesdites séquences sont délivrées à des zones corporelles d'un organisme indépendamment pour chaque canal;
- au moins deux électrodes de stimulation (1, 2, 102, 105), dont chacune est connectée ou peut être connectée à un canal de stimulation respectif et dont chacune est destinée à être appliquée de manière amovible à l'une de deux zones corporelles prédéfinies différentes d'un organisme;
- chaque électrode (1, 2, 102, 105) étant appliquée extérieurement sur la peau du patient en correspondance de ladite zone prédéfinie et avec une relation de position prédéfinie par rapport à la position de l'électrode ou des électrodes restantes;
- une unité de commande (14; 130) dudit au moins un générateur d'impulsions électriques (111) communiquant avec ledit un générateur d'impulsions électriques;
- une interface de commande et/ou de saisie de données pour régler et/ou afficher des données de réglage et/ou régler des paramètres dudit un ou desdits plusieurs générateurs (111),
- ledit pansement étant constitué d'un film comprenant une solution de povidone iodée (PVP-I) et d'alginate de sodium (NaAlg) et étant destiné à être appliqué lors du traitement de l'ulcère;
et dans lequel
- les au moins deux électrodes (1, 2, 102, 105) sont destinées à être disposées sur des côtés diamétralement opposés de la lésion à une distance prédéfinie de celle-ci.

2. Le dispositif selon la revendication 1, dans lequel la distance entre chaque position de chaque électrode (1, 2, 102, 105) appliquée sur le corps du patient et le bord de la lésion ou de l'ulcère faisant face à l'électrode correspondante est de l'ordre d'au moins quelques centimètres, notamment d'au moins 1cm.

3. Le dispositif selon la revendication 1 ou 2, dans lequel le pansement est constitué d'une solution d'Alginate de Sodium, de PVP-I et de glycérol.

4. Le dispositif selon l'une ou plusieurs des revendications précédentes, dans lequel le pansement est constitué d'un film obtenu en ajoutant à une solution comprenant de l'alginate de sodium à une concentration de 4,6 à 5,2% p/v et du PVP-I à une concentration de 1,3 à 1,9% p/v et du glycérol à une concentration de 1,3 à 2,3 p/v par rapport au poids total d'alginate de sodium et de PVP-I.

5. Le dispositif selon l'une ou plusieurs des revendications précédentes, dans lequel lesdites au moins deux électrodes (1, 2, 102, 105) sont chacune portées par un élément de support auto-adhésif flexible indépendant (10, 11; 150; 600) qui peut être fixé dans une position prédéfinie par rapport à l'anatomie du corps humain.

6. Le dispositif selon l'une ou plusieurs des revendications précédentes 1 à 4, dans lequel lesdites au moins deux électrodes (1, 2, 102, 105) sont portées par un seul élément de support auto-adhésif flexible (150) qui peut être fixé dans une position prédéfinie par rapport à l'anatomie du corps humain, ledit élément flexible ayant au moins deux branches qui délimitent une zone au moins de forme concave pour loger la lésion, tandis que lesdites électrodes sont chacune prévues sur une extrémité de la branche respective, ladite zone concave étant configurée pour loger un film de pansement.

7. Le dispositif selon l'une ou plusieurs des revendications 5 et 6, dans lequel ledit élément flexible est sous la forme d'un cadre fermé sur lui-même ayant une zone centrale libre destinée à recevoir la lésion, lesdites au moins deux électrodes (1, 2, 102, 105) étant prévues sur ledit cadre en correspondance de deux points de celui-ci diamétralement opposés l'un par rapport à l'autre par rapport au centre dudit cadre, tandis que le pansement est constitué d'un film de forme et de dimension telles qu'il peut au moins être inscrit à l'intérieur dudit cadre.

8. Le dispositif selon l'une ou plusieurs des revendications précédentes 5 à 7, dans lequel une couche de fermeture est prévue, qui recouvre, au moins partiellement, le cadre et/ou les branches de support des électrodes (1, 2, 102, 105) ainsi que la zone concave ou la zone centrale, adhérant uniquement à l'un ou aux éléments de support flexibles (150) des électrodes et donc au cadre et/ou aux deux branches de support des électrodes et n'ayant pas d'action adhésive en correspondance de la lésion ou du film de pansement.

9. Le dispositif selon l'une ou plusieurs des revendications précédentes, dans lequel est prévue une série d'éléments flexibles (10, 11; 150; 600) pour supporter les électrodes (1, 2, 102, 105) et/ou le film de pansement de formes et/ou de dimensions destinées à des lésions ayant des formes et/ou des dimensions différentes.

10. Le dispositif selon l'une ou plusieurs des revendications précédentes, dans lequel le générateur (111) précité, l'unité de commande (14; 130) précitée et l'interface de commande et/ou d'affichage (17, 18) précitée(s) et la source d'alimentation (13) de ces composants sont intégrés dans un seul dispositif de commande (106; 550; 610), ledit dispositif étant connecté ou pouvant être connecté au moyen de câbles électriques (107, 108) aux électrodes (1, 2, 102, 105) précitées et ladite connexion pouvant être fixe, ou la connexion pouvant être réalisée au moyen de bornes de couplage prévues à l'extrémité du câble de connexion à l'électrode correspondante, qui sont connectées de manière séparable à des bornes de connexion correspondantes prévues avec ladite électrode correspondante.

11. Le dispositif selon l'une ou plusieurs des revendications précédentes, dans lequel le dispositif de commande (106) est prévu en combinaison avec un élément portable pour la fixation amovible dudit dispositif de commande à une région anatomique du patient.

12. Le dispositif selon la revendication 11, dans lequel lesdits éléments portables sont pourvus de poches de logement pouvant être fermées et ouvertes pour ledit dispositif de commande (106).

13. Le dispositif selon les revendications 11 ou 12, dans lequel lesdits éléments portables, sous la forme d'éléments flexibles, sont constitués d'une ou plusieurs extensions latérales externes d'une branche des au moins deux branches de l'élément de support flexible des électrodes (1, 2, 102, 105) ou de l'élément de support flexible sous forme d'un cadre.

14. Le dispositif selon l'une ou plusieurs des revendications précédentes 5-9 ou 13, dans lequel des conducteurs connectant les électrodes au canal correspondant du générateur sont intégrés dans la structure de l'élément flexible.

15. Le dispositif selon l'une ou plusieurs des revendications précédentes 5-9 ou 13-14, dans lequel un élément de support du dispositif de commande (106) a une borne pour connecter des conducteurs connectant les électrodes au dispositif de commande, qui est constituée d'une base de connexion fixée de manière stable audit élément flexible.

16. Le dispositif selon l'une ou plusieurs des revendications précédentes 5-9 ou 13-15, dans lequel ledit élément flexible est façonné de manière à délimiter une zone concave et/ou une fenêtre entourée d'un cadre fermé sur lui-même et comprenant au moins un élément d'un film ou une pluralité d'éléments dudit film de pansement ayant une forme sensiblement congruente à la zone délimitée par l'élément de support flexible, ledit dispositif de commande (106) étant appliqué de manière amovible audit élément flexible et une feuille de couverture de la zone concave et/ou de ladite fenêtre étant éventuellement incluse et pouvant être appliquée de manière amovible sur la face opposée à la surface d'application de l'élément flexible et qui recouvre au moins partiellement ladite zone concave et/ou ladite fenêtre et/ou ledit dispositif de commande.

17. Un kit pour le traitement thérapeutique d'ulcères, dans lequel la combinaison d'un élément de support flexible pour deux électrodes (1, 2, 102, 105) et d'un support pour au moins un dispositif de commande (106) et au moins un film de pansement comprend au moins un élément flexible et un dispositif selon l'une ou plusieurs des revendications 1 à 16.
